# FASCICULE DE BREVET EUROPEEN

(11) **EP 1 585 466 B1**
(45) Date de publication et mention de la délivrance du brevet: **22.08.2007**
(21) Numéro de dépôt: 03815408.4
(22) Date de dépôt: 16.12.2003
(51) Int. Cl.: A61F 2/44

(54) **IMPLANT INTERSOMATIQUE POUR VERTEBRES**
BANDSCHEIBENIMPLANTAT
INTERVERTEBRAL IMPLANT

(30) Priorité: 17.12.2002 FR 0216234
(43) Date de publication de la demande: 19.10.2005
(73) Titulaire: Vitatech, F-74970 Marignier (FR)
(72) Inventeur: GRADEL, Thomas, F-74130 Ayze (FR); LEMAIRE, Jean-Philippe, Le Pré Loiseau, F-21910 Saulon La Chapelle (FR)
(74) Mandataire: Poncet, Jean-François
(86) Numéro de dépôt international: PCT/FR2003/003734
(87) Numéro de publication internationale: WO 2004/064691

(56) Documents cités:
- WO-A-02/15825
- WO-A-97/31517

## Description

### DOMAINE TECHNIQUE DE L'INVENTION

La présente invention concerne les implants intersomatiques creux pour stabilisation de la colonne vertébrale, destinés à former une cale à insérer entre les plateaux en vis à vis de deux vertèbres adjacentes, de manière à maintenir un espace discal constant. Le document WO 97/31517 décrit un implant selon le préambule de la revendication 1.

Le document EP 0 307 241 A décrit un exemple d'implant intersomatique ayant la forme d'une cage parallélépipédique. Une telle forme de cage n'est pas bien adaptée à une utilisation dans la colonne vertébrale, notamment par le fait que les arêtes longitudinales d'une telle cage risquent de léser la moelle épinière ou les racines lors de l'insertion de l'implant entre les vertèbres adjacentes. On constate également une relative inefficacité du maintien des vertèbres adjacentes, vraisemblablement à cause de la forme parallélépipédique de la cage.

On a également proposé un implant intersomatique de forme parallélépipédique dans lequel les grandes faces supérieure et inférieure sont planes et forment entre elles un angle ouvert vers l'avant, pour maintenir les vertèbres adjacentes selon un angle physiologique approprié de lordose. La forme de l'implant nécessite de réaliser des évidements conséquents dans les plateaux vertébraux, pour permettre l'insertion de l'implant par translation antéropostérieure lors de la pose, ce qui crée un traumatisme inapproprié.

Le document US 5 888 227 A décrit un implant intersomatique à paroi périphérique entourant une grande cavité intérieure unique ouverte à l'avant et à l'arrière. Les faces supérieure et inférieure sont divergentes vers l'avant et présentent une double courbure antéro-postérieure et transversale. La structure présente une résistance mécanique insuffisante, et la tenue des vertèbres adjacentes est insuffisante.

Le document WO 02/15825 A décrit un implant intersomatique de forme générale cylindrique, avec des parois supérieure et inférieure munies chacune de deux orifices, avec une paroi antérieure à trou taraudé pour la manipulation par un outil de pose, et avec des parois latérales ayant chacune une rainure longitudinale dont les bords s'opposeraient à une rotation axiale de l'implant lors de la pose.

### EXPOSE DE L'INVENTION

Le problème proposé par la présente invention est de définir une nouvelle structure d'implant intersomatique qui permette de réduire très sensiblement les risques de lésions nerveuses lors de la pose, qui permette d'assurer un meilleur positionnement et un meilleur blocage des vertèbres l'une avec l'autre, et qui minimise le traumatisme osseux lors de la pose. On cherche notamment à assurer une résistance mécanique améliorée et un bon accrochage osseux en facilitant la fusion entre deux vertèbres adjacentes.

Pour atteindre ces buts ainsi que d'autres, l'implant intersomatique selon l'invention, permettant la stabilisation de vertèbres adjacentes, comprend un corps d'implant en matériau solide biocompatible ayant une structure générale tubulaire limitée par deux parois supérieure et inférieure convexes légèrement divergentes vers l'avant, par deux parois latérales planes opposées , et par une paroi postérieure à trou axial avec une cavité intérieure unique mettant en communication des orifices prévus dans les parois supérieure et inférieure, dans lequel :
- chaque paroi supérieure et inférieure comprend un orifice respectif supérieur ou inférieur unique largement dimensionné,
- le trou axial est taraudé,
- un bouchon interchangeable de compression est adaptable par vissage dans le trou axial taraudé de la paroi postérieure,
- la cavité intérieure est fermée vers l'avant par une paroi antérieure,
- la largeur de l'implant, définie par les parois latérales, est inférieure à sa hauteur définie par les parois supérieure et inférieure,
- les parois latérales, planes sont légèrement divergentes vers l'avant.

Une telle structure à grande cavité centrale et orifices largement ouverts dans la paroi supérieure et dans la paroi inférieure favorise le contact entre un greffon inséré à l'intérieur du corps d'implant et les deux plateaux vertébraux entre lesquels est inséré l'implant intersomatique. La mise en place du greffon est facilitée. Le bouchon interchangeable de compression permet de comprimer le greffon contre les deux vertèbres adjacentes après la mise en place. Enfin, l'implant peut être mis en position entre les deux vertèbres en étant pivoté de 90° pour que ses faces latérales plus rapprochées l'une de l'autre se trouvent au-dessus et au-dessous, face aux plateaux vertébraux des deux vertèbres adjacentes. On effectue ensuite une rotation de 90° afin de mettre le greffon en contact avec les vertèbres. On réduit ainsi le traumatisme de la zone vertébrale, tout en minimisant la quantité de matière qu'il faut enlever pour engager l'implant intersomatique entre les vertèbres adjacentes.

Selon un mode de réalisation avantageux, chaque orifice supérieur ou inférieur ouvre la cavité intérieure sur toute sa largeur entre les parois latérales et sur toute sa longueur entre les parois postérieure et antérieure. On facilite ainsi la mise en place du greffon, et on maximise son contact avec les vertèbres adjacentes.

De préférence, le bouchon interchangeable de compression comprend un tronçon d'extrémité intérieur conique. Cela facilite la pénétration du bouchon dans le greffon, pour sa compression.

Pour obtenir une compression efficace, on peut avantageusement prévoir que le bouchon interchangeable de compression, et le trou axial taraudé qui le reçoit, ont un diamètre sensiblement égal à la largeur de la cavité intérieure au voisinage de la paroi postérieure.

De bons résultats sont obtenus en prévoyant que le bouchon interchangeable de compression a une longueur telle que, en fin de vissage dans le trou axial taraudé qui le reçoit, son tronçon d'extrémité intérieur pénètre dans la cavité intérieure selon une longueur d'au moins le quart de la longueur de ladite cavité intérieure.

De préférence, on prévoit au moins deux bouchons interchangeables de compression ayant des longueurs différentes, de sorte que le praticien peut choisir le bouchon le mieux adapté à la compression du greffon.

Selon un mode de réalisation avantageux, la paroi postérieure du corps d'implant comporte une rainure diamétrale externe pour permettre l'actionnement de l'implant en rotation axiale, afin de le faire passer depuis une position d'introduction, avec ses parois latérales en positions haute et basse, jusqu'à une orientation de soutien des vertèbres avec les parois supérieure et inférieure en appui sur les plateaux vertébraux adjacents.

On peut réaliser l'implant en un matériau biocompatible solide tel que le titane, ou avantageusement en un polymère de type PEEK, qui présente l'avantage d'être radiotransparent.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles:
- la figure 1 est une vue en perspective de 3/4 arrière d'un corps d'implant intersomatique selon un mode de réalisation de la présente invention ;
- la figure 2 est une vue en perspective de 3/4 avant du corps d'implant de la figure 1 ;
- les figures 3 à 6 illustrent respectivement, en vue de dessus, en vue de côté, en vue d'avant et en vue d'arrière, la structure de corps d'implant des figures 1 et 2 ;
- la figure 7 est une vue en perspective de 3/4 arrière d'un implant selon l'invention, comprenant le corps d'implant de la figure 1 et un bouchon interchangeable de compression ; et
- la figure 8 est une vue de dessus de l'implant de la figure 7.

### DESCRIPTION DES MODES DE REALISATION PREFERES

Dans le mode de réalisation illustré sur les figures, l'implant intersomatique selon l'invention comprend un corps d'implant 1 en matériau solide biocompatible, qui présente une structure générale tubulaire limitée par deux grandes parois supérieure 2 et inférieure 3 convexes légèrement divergentes vers l'avant, par deux parois latérales opposées 4 et 5 planes légèrement divergentes vers l'avant, par une paroi postérieure 6 à trou axial taraudé 7, et par une paroi antérieure 8.

L'intérieur du corps d'implant 1 est une cavité intérieure 9 unique, ouverte vers le haut par un orifice supérieur 10, et ouverte vers le bas par un orifice inférieur 11. La cavité intérieure 9 met ainsi en communication les orifices supérieur 10 et inférieur 11 prévus dans les grandes faces supérieure 2 et inférieure 3 du corps d'implant 1.

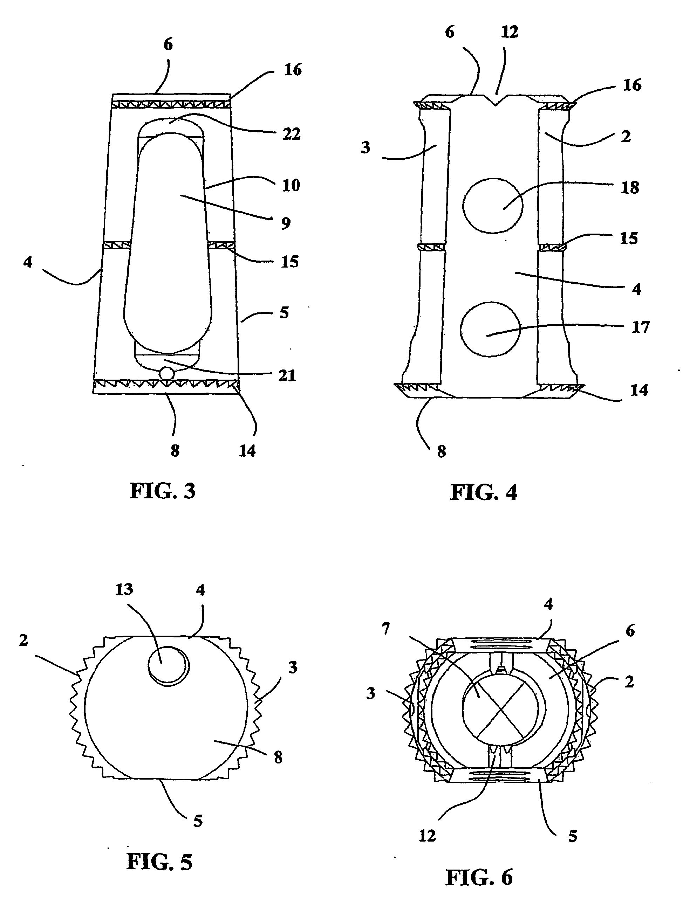

Dans la réalisation illustrée sur les figures, comme on le voit mieux notamment sur la figure 3, la cavité intérieure 9 a une forme générale en tronc de pyramide qui va en s'évasant vers la paroi antérieure 8. L'orifice supérieur 10 et l'orifice inférieur 11 ouvrent la cavité intérieure 9 sur toute sa largeur entre les parois latérales 4 et 5 et sur toute sa longueur entre les parois postérieure 6 et antérieure 8.

La paroi postérieure 6 comporte une rainure diamétrale externe 12, qui permet d'engager un outil telle qu'une lame de tournevis pour imprimer au corps d'implant un mouvement de rotation axiale.

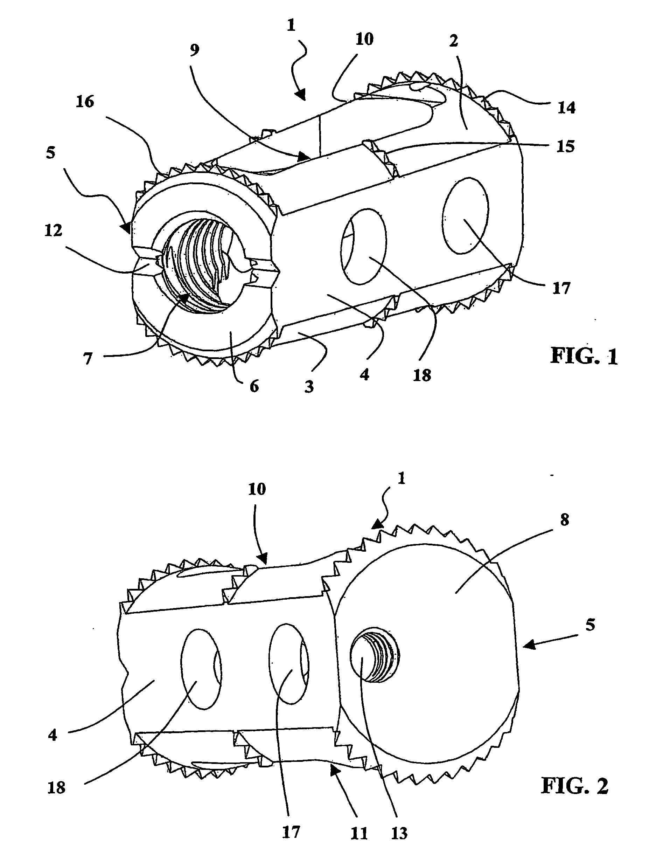

La paroi antérieure 8 comporte un trou taraudé excentré 13, comme illustré sur la figure 2 ou la figure 5, le trou taraudé excentré 13 ayant un diamètre nettement plus petit que le diamètre de la paroi antérieure 8.

Les grandes parois supérieure 2 et inférieure 3 comportent des nervures annulaires dentées anti-expulsion, telles que les nervures antérieure 14, médiane 15 et postérieure 16.

Les parois latérales telles que la paroi 4 peuvent comprendre de petites ouvertures latérales telles que les trous 17 et 18.

Comme on le voit notamment sur les figures 5 et 6, la largeur du corps d'implant, ou distance entre les faces externes de ses parois latérales 4 et 5, est inférieure à la hauteur de l'implant ou distance définie par les faces externes de ses parois supérieure 2 et inférieure 3.

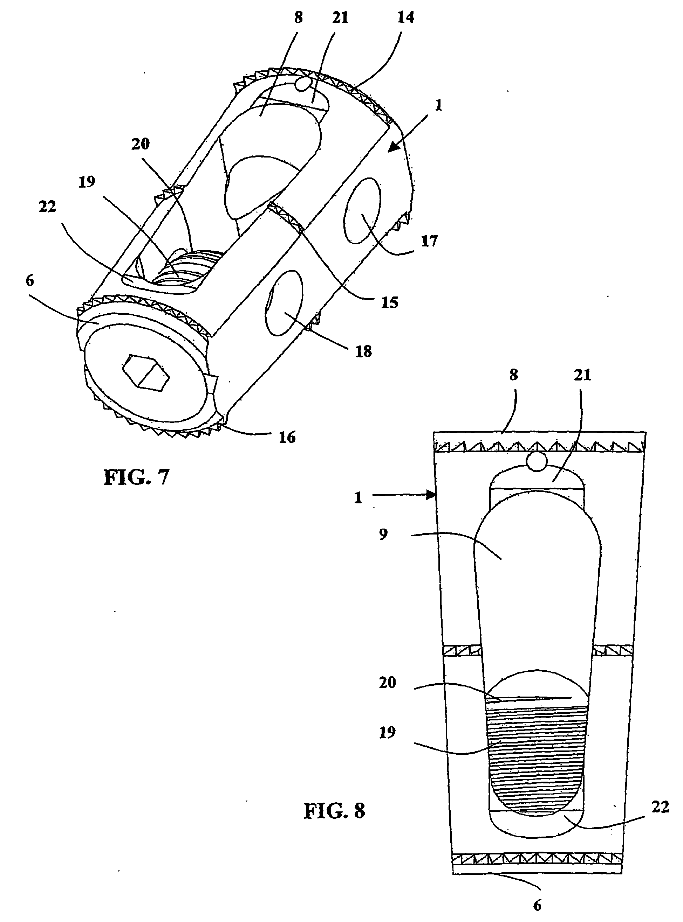

En considérant maintenant les figures 7 et 8, qui illustrent l'implant intersomatique selon l'invention à l'état assemblé, on retrouve le corps d'implant 1 tel que décrit en relation avec les figures 1 à 6, et l'on voit que l'implant comporte en outre un bouchon interchangeable de compression 19, adapté par vissage dans le trou axial taraudé 7 de la paroi postérieure 6.

Le bouchon interchangeable de compression 19 présente, tout comme le trou axial taraudé 7, un diamètre sensiblement égal à la plus petite largeur de la cavité intérieure 9 ou distance séparant les parois latérales 4 et 5 au voisinage de la paroi postérieure 6.

Dans la réalisation illustrée sur les figures 7 et 8, le bouchon interchangeable de compression 19 comprend un tronçon d'extrémité 20 intérieur généralement conique ou en ogive, facilitant sa pénétration dans un greffon inséré dans la cavité intérieure 9.

Le bouchon interchangeable de compression 19 peut être réalisé en titane, pour être repérable par radio. Par contre, le corps d'implant 1 peut avantageusement être en un matériau radiotransparent, avantageusement en polymère de type PEEK (polyétheréthercétone).

Dans ce cas, on peut avantageusement prévoir un marqueur en titane, disposé dans le corps d'implant 1 à l'écart du bouchon interchangeable de compression 19.

Dans la réalisation illustrée sur les figures, chaque paroi supérieure 2 et inférieure 3 est de forme générale conique, et chaque orifice supérieur 10 ou inférieur 11 est bordé, à ses extrémités antérieure et postérieure, par un plat respectif 21 ou 22 perpendiculaire aux parois latérales 4 et 5.

Pour la mise en place de l'implant selon l'invention entre deux vertèbres adjacentes à traiter, on peut procéder selon les étapes ci-après :
- on avive les zones de plateaux entre lesquelles doit se loger l'implant,
- on loge un greffon osseux dans la cavité intérieure 9 du corps d'implant 1,
- on oriente le corps d'implant 1 pour placer ses faces planes latérales 4 et 5 parallèlement aux plateaux de vertèbres à traiter, de façon que l'implant présente une hauteur minimale,
- on engage le corps d'implant 1 ainsi orienté entre les deux vertèbres adjacentes à traiter maintenues écartées,
- on fait pivoter axialement le corps d'implant 1 de 90°, pour placer ses parois supérieure 2 et inférieure 3 contre les plateaux de vertèbres à traiter, puis on relâche les vertèbres,
- on visse le bouchon interchangeable de compression 19 pour comprimer le greffon et le presser ainsi contre les plateaux de vertèbres à traiter.

La présente invention n'est pas limitée aux modes de réalisation qui ont été explicitement décrits, mais elle en inclut les diverses variantes et généralisations contenues dans le domaine des revendications ci-après.

## Revendications

1. - Implant intersomatique pour stabilisation de vertèbres adjacentes, comprenant un corps d'implant (1) en matériau solide biocompatible ayant une structure générale tubulaire limitée par deux parois supérieure (2) et inférieure (3) convexes légèrement divergentes vers l'avant, par deux parois latérales opposées (4, 5) planes, et par une paroi postérieure (6) à trou axial (7), avec une cavité intérieure (9) unique mettant en communication des orifices prévus dans les parois supérieure (2) et inférieure (3), dans lequel :
- chaque paroi supérieure (2) et inférieure (3) comprend un orifice respectif supérieur (10) ou inférieur (11) unique largement dimensionné,
- la cavité intérieure (9) est fermée vers l'avant par une paroi antérieure (8), et
- la largeur de l'implant, définie par les parois latérales (4, 5), est inférieure à sa hauteur définie par les parois supérieure (2) et inférieure (3),
**caractérisé en ce que** :
les parois latérales (4, 5) sont légèrement divergentes vers l'avant,
le trou axial (7) est taraudé,
et un bouchon interchangeable de compression (19) est adaptable par vissage dans le trou axial taraudé (7) de la paroi postérieure (6).

2. - Implant selon la revendication 1, **caractérisé en ce que** chaque orifice supérieur (10) ou inférieur (11) ouvre la cavité intérieure (9) sur toute sa largeur entre les parois latérales (4, 5) et sur toute sa longueur entre les parois postérieure (6) et antérieure (8).

3. - Implant selon l'une des revendications 1 ou 2, **caractérisé en ce que** le bouchon interchangeable de compression (19) comprend un tronçon d'extrémité (20) intérieur conique.

4. - Implant selon l'une quelconque des revendications 1 à 3, **caractérisé en ce que** le bouchon interchangeable de compression (19) et le trou axial taraudé (7) qui le reçoit, ont un diamètre sensiblement égal à la largeur de la cavité intérieure (9) au voisinage de la paroi postérieure (6).

5. - Implant selon l'une quelconque des revendications 1 à 4, **caractérisé en ce que** le bouchon interchangeable de compression (19) a une longueur telle que, en fin de vissage dans le trou axial taraudé (7) qui le reçoit, son tronçon d'extrémité intérieur (20) pénètre dans la cavité intérieure (9) selon une longueur d'au moins le quart de la longueur de ladite cavité intérieure (9).

6. - Implant selon l'une quelconque des revendications 1 à 5, **caractérisé en ce qu'**on prévoit au moins deux bouchons interchangeables de compression (19) ayant des longueurs différentes.

7. - Implant selon l'une quelconque des revendications 1 à 6, **caractérisé en ce que** la paroi postérieure (6) du corps d'implant (1) comporte une rainure diamétrale externe (12) pour actionnement de l'implant en rotation axiale.

8. - Implant selon l'une quelconque des revendications 1 à 7, **caractérisé en ce que** la paroi antérieure (8) comporte un trou taraudé excentré (13) de plus petit diamètre.

9. - Implant selon l'une quelconque des revendications 1 à 8, **caractérisé en ce que** les grandes parois supérieure (2) et inférieure (3) comportent des nervures (14, 15, 16) annulaires dentées anti-expulsion.

10. - Implant selon l'une quelconque des revendications 1 à 9, **caractérisé en ce que** le bouchon interchangeable de compression (19) est en titane.

11. - Implant selon l'une quelconque des revendications 1 à 10, **caractérisé en ce que** le corps de l'implant (1) est en un polymère de type PEEK.

12. - Implant selon la revendication 11, **caractérisé en ce qu'**il comprend un marqueur en titane, disposé dans le corps d'implant (1) à l'écart du bouchon interchangeable de compression (19).

13. - Implant selon l'une quelconque des revendications 1 à 12, **caractérisé en ce que** chaque paroi supérieure (2) et inférieure (3) est de forme générale conique, et chaque orifice supérieur (10) et inférieur (11) est bordé, à ses extrémités antérieure et postérieure, par un plat (21, 22) perpendiculaire aux parois latérales (4, 5).

## Claims

1. Intervertebral implant for stabilizing adjacent vertebrae, comprising a solid biocompatible material implant body (1) having a tubular general structure delimited by an upper wall (2) and a lower wall (3) that are convex and slightly divergent toward the front, two opposite lateral walls (4, 5) that are plane, and a posterior wall (6) with a axial hole (7), with a single interior cavity (9) providing communication between orifices provided in the upper wall (2) and the lower wall (3), wherein :
- the upper wall (2) and the lower wall (3) each comprise a respective single large upper orifice (10) or lower orifice (11),
- the interior cavity (9) is closed toward the front by an anterior wall (8), and
- the width of the implant defined by the lateral walls (4, 5) is less than its height defined by the upper wall (2) and the lower wall (3),
**characterized in that** :
the lateral walls (4, 5) are slightly divergent toward the front,
the axial hole (7) is threaded,
and an interchangeable compression plug (19) is adapted to be fitted by screwing it into the threaded axial hole (7) in the posterior wall (6).

2. Implant according to claim 1, **characterized in that**, because of the upper orifice (10) and the lower orifice (11), the interior cavity (9) is open over the whole of its width between the lateral walls (4, 5) and over the whole of its length between the posterior wall (6) and the anterior wall (8).

3. Implant according to either claim 1 or claim 2, **characterized in that** the interchangeable compression plug (19) comprises a conical interior end portion (20).

4. Implant according to any one of claims 1 to 3, **characterized in that** the interchangeable compression plug (19) and the threaded axial hole (7) that receives it have a diameter substantially equal to the width of the interior cavity (9) in the vicinity of the posterior wall (6).

5. Implant according to any one of claims 1 to 4, **characterized in that** the interchangeable compression plug (19) has a length such that, at the end of screwing it into the threaded axial hole (7) that receives it, its interior end portion (20) penetrates the interior cavity (9) to a distance of at least one quarter of the length of said interior cavity (9).

6. Implant according to any one of claims 1 to 5, **characterized in that** it is provided with at least two interchangeable compression plugs (19) having different lengths.

7. Implant according to any one of claims 1 to 6, **characterized in that** the posterior wall (6) of the implant body (1) includes an external diametral groove (12) for actuating axial rotation of the implant.

8. Implant according to any one of claims 1 to 7, **characterized in that** the anterior wall (8) includes an eccentric threaded hole (13) of smaller diameter.

9. Implant according to any one of claims 1 to 8, **characterized in that** the upper larger wall (2) and the lower larger wall (3) include annular toothed anti-expulsion ribs (14, 15, 16).

10. Implant according to any one of claims 1 to 9, **characterized in that** the interchangeable compression plug (19) is made of titanium.

11. Implant according to any one of claims 1 to 10, **characterized in that** the implant body (1) is made of a PEEK type polymer.

12. Implant according to claim 11, **characterized in that** it comprises a titanium marker in the implant body (1) away from the interchangeable compression plug (19).

13. Implant according to any one of claims 1 to 12, **characterized in that** the upper wall (2) and lower wall (3) are each of conical general shape and the upper orifice (10) and lower orifice (11) are each bordered at their anterior and posterior ends by a flat (21, 22) perpendicular to the lateral walls (4, 5).

## Patentansprüche

1. Intervertebrales Implantat zur Stabilisierung benachbarter Wirbel, mit einem Implantatkörper (1) aus festem biokompatiblem Material, der eine allgemein röhrenförmige Struktur hat, die durch zwei Wände, eine obere Wand (2) und eine untere Wand (3), die konvex sind und leicht nach vorne divergieren, durch zwei gegenüberliegende ebene Seitenwände (4, 5) und durch eine hintere Wand (6) mit einem axialen Loch (7) begrenzt ist, mit einem einzigen Innenraum (9), der eine Verbindung zwischen der oberen Wand (2) und der unteren Wand (3) vorgesehenen Öffnungen herstellt, wobei:
- jede obere Wand (2) und untere Wand (3) eine entsprechende einzige obere Öffnung (10) oder untere Öffnung (11) von reichlicher Dimension aufweist,
- der Innenraum (9) nach vorne durch eine Vorderwand (8) verschlossen ist, und
- die Breite des Implantats, die durch die Seitenwände (4, 5) definiert ist, kleiner ist als seine Höhe, die durch die obere (2) und untere (3) Wand definiert ist,
**dadurch gekennzeichnet, daß**:
die Seitenwände (4, 5) leicht nach vorne divergieren,
das axiale Loch (7) ein Gewinde aufweist, und
ein auswechselbarer Kompressionszapfen (19) zum Einschrauben in das axiale Gewindeloch (7) der hinteren Wand (6) ausgebildet ist.

2. Implantat nach Anspruch 1, **dadurch gekennzeichnet, daß** jede der oberen (10) oder unteren (11) Öffnung den Innenraum (9) über dessen gesamte Breite zwischen den Seitenwänden (4, 5) und über dessen gesamte Länge zwischen der hinteren Wand (6) und der hinteren Wand (8) öffnet.

3. Implantat nach einem der Ansprüche 1 oder 2, **dadurch gekennzeichnet, daß** der auswechselbare Kompressionszapfen (19) ein inneres konisches Endteil (20) aufweist.

4. Implantat nach irgendeinem der Ansprüche 1 bis 3, **dadurch gekennzeichnet, daß** der auswechselbare Kompressionszapfen (19) und das axiale Gewindeloch (7), das ihn aufnimmt, einen Durchmesser haben, der im wesentlichen gleich der Breite des Hohlraumes (9) in der Nähe der hinteren Wand (6) ist.

5. Implantat nach irgendeinem der Ansprüche 1 bis 4, **dadurch gekennzeichnet, daß** der auswechselbare Kompressionszapfen (19) eine Länge hat, die derart ist, daß am Ende des Einschraubens in das axiale Loch (7), welches ihn aufnimmt, sein inneres Endteil (20) in den Innenraum (9) mit einer Länge eindringt, die mindestens ein Viertel der Länge des genannten Innenraumes (9) beträgt.

6. Implantat nach irgendeinem der Ansprüche 1 bis 5, **dadurch gekennzeichnet, daß** mindestens zwei auswechselbare Kompressionszapfen (19) mit unterschiedlichen Längen vorgesehen sind.

7. Implantat nach irgendeinem der Ansprüche 1 bis 6, **dadurch gekennzeichnet, daß** die hintere Wand (6) des Implantatkörpers (1) eine äußere diametrale Nut (12) aufweist, zum Bewirken einer axialen Drehung des Implantates.

8. Implantat nach irgendeinem der Ansprüche 1 bis 7, **dadurch gekennzeichnet, daß** die vordere Wand (8) ein exzentrisches Gewindeloch (13) mit kleinerem Durchmesser aufweist.

9. Implantat nach irgendeinem der Ansprüche 1 bis 8, **dadurch gekennzeichnet, daß** die großen oberen (2) und unteren (3) Wände ringförmige gezahnte Anti-Entfernungs-Rippen (14, 15, 16) aufweisen.

10. Implantat nach irgendeinem der Ansprüche 1 bis 9, **dadurch gekennzeichnet, daß** der auswechselbare Kompressionszapfen (19) aus Titan ist.

11. Implantat nach irgendeinem der Ansprüche 1 bis 10, **dadurch gekennzeichnet, daß** der Implantatkörper (1) aus einem Polymer des Typs PEEK ist.

12. Implantat nach Anspruch 11, **dadurch gekennzeichnet, daß** er eine Titanmarkierung aufweist, die im Implantatkörper (1) entfernt vom auswechselbaren Kompressionszapfen (19) angeordnet ist.

13. Implantat nach irgendeinem der Ansprüche 1 bis 12, **dadurch gekennzeichnet, daß** jede der oberen (2) und untern (3) Wände generell von konischer Form ist, und jede der oberen (10) und unteren (11) Öffnungen an ihren vorderen und hinteren Enden durch ein ebenes Teil (21, 22), das senkrecht zu den Seitenwänden (4, 5) verläuft, eingefasst ist.
